# EUROPEAN PATENT APPLICATION

(11) **EP 4 368 214 A1**
(43) Date of publication of application: **15.05.2024**
(21) Application number: 22211646.9
(22) Date of filing: 06.12.2022
(51) Int. Cl.: A61L 27/42, B33Y 10/00, B33Y 80/00

(54) **INTELLIGENT THREE-DIMENSIONAL BIOMIMETIC STRUCTURE AND USE THEREOF**

(30) Priority: 14.11.2022 PT 2022118343
(71) Applicant: Instituto Politécnico De Leiria, 2411-901 Leiria (PT)
(72) Inventor: FERNANDES PATRÍCIO, TATIANA MARISA, 2430-028 MARINHA GRANDE (PT); DOS SANTOS MATEUS, ARTUR JORGE, 2430-028 MARINHA GRANDE (PT); FERNANDES ALVES, NUNO MANUEL, 2430-028 MARINHA GRANDE (PT); REIS MARQUES, HUGO FILIPE, 2430-028 MARINHA GRANDE (PT)
(74) Representative: Monteiro Alves, Inês

(57) **Abstract**

The invention describes an intelligent three-dimensional biomimetic structure and the use thereof in the regeneration of bone tissue after a trauma or cancer situation.

Said intelligent three-dimensional biomimetic structure is comprised of filaments constituted by polymers mineralized with hydroxyapatite, which are able to mimic the bone tissue in terms of composition, morphology, and mechanical properties.

## Description

### TECHNICAL DOMAIN

The present invention refers to an intelligent three-dimensional biomimetic structure and the use thereof for the regeneration of bone tissue.

### PRIOR ART

Bone defects cause a reduction in the quality of life and can result from countless factors, such as traumas, degenerative diseases, oncological diseases, natural aging process or obesity.

Several commercially available devices help to remedy the diseases associated with bone degeneration, however, many times they cause adverse effects which can extend the treatment, leading to increase in costs for the health system in question.

This situation can be minimized by means of the use of a three-dimensional model which presents biomimetic properties that accompany the regeneration process.

In light of the above-mentioned context, studies have been developed with the purpose of conceiving alternative technologies which can be useful in the treatment of diseases associated with bone degeneration. To this end, different three-dimensional models, or scaffolds are already known from the state of the art.

Document CN111110404A describes a bone composite scaffold comprising a multi-layer structure. The layers are made of composite materials in different proportions, different 3D printing fiber spacings and porosity factors, to resemble a real bone structure. The bone repair effect is promoted by adding metallic ions and performing surface modification treatment.

Document EP3266418B1 refers to an alloprosthetic composite implant comprising a porous structure having a pore density profile corresponding to a bone. A plurality of cells, such as osteoblasts and / or stem cells, are seeded within the pores and grown by incubation.

Document EP2349107B1 refers to customized implants comprised of polymers (such as the poly [ether ketone ketone]) for the bone replacement. These implants present mechanical properties similar (if not identical) to that of natural bone and are prepared by computer-based imaging and rapid prototyping (RP).

In document EP3563881A1, there is provided a method for producing a synthetic bone graft by 3D printing, as well as the grafts produced. Said synthetic bone graft comprises calcium-deficient hydroxyapatite (CDHA) in an amount of at least 60% by weight relative to the total weight of the composition.

Differently from these documents, this invention proposes an intelligent three-dimensional biomimetic structure presenting morphological, physical, thermal, mechanical, chemical, and biological properties which mimic the bone tissue.

Said intelligent three-dimensional biomimetic structure comprises a polymer matrix and an inorganic matrix, which create a three-dimensional endogenous environment and facilitator in cell recognition, subsequently to the implantation process, without the need for cell implantation.

The polymer matrix is comprised by synthetic or natural polymers, whereby these are mineralized with hydroxyapatite (which, in turn, can be doped with several ions).

The doped hydroxyapatite used in the present invention imparts biomimetic and magnetic properties to the intelligent three-dimensional biomimetic structure. Further, the use of several ions will allow the structures to have antimicrobial and therapeutic properties that are suitable for the intended use, whereby they may be used in the prevention, diagnosis and treatment of diseases associated with the skeletal system.

The article "*Bio-inspired polymeric iron-doped hydroxyapatite microspheres as a tunable carrier of rhBMP-2*" demonstrates the ability of superparamagnetic microspheres having a similar composition to that of the bone in capturing and distributing recombinant human bone morphogenetic protein-2 (rhBMP-2) in therapeutically relevant doses, apart from the osteogenic properties thereof, which can be observed by the presence of three human mesenchymal cells.

In the article "*Superparamagnetic hybrid microspheres affecting osteoblasts behavior*", the study carried out demonstrates that, in the presence of calcium and iron ions originating from hybrid microspheres in a culture which mimics the physiological environment, the osteoblasts present cell viability and differentiation.

The article "*New bioactive bone-like microspheres with intrinsic magnetic properties obtained by bio-inspired mineralisation process*" presents the method for obtaining hybrid magnetic particles by biomineralization followed by emulsification and hydrothermal stabilization.

In this context, the present invention arises as a therapeutic alternative for the regeneration of bone tissue, particularly after a trauma or cancer situation.

### PROBLEMS OF THE PRIOR ART

The majority of the studies and publications relating to three-dimensional structures or scaffolds demonstrate the possibility of development of three-dimensional matrices with synthetic polymer materials and also by adding ceramic and metallic materials, to grant the necessary mechanical properties to bear the strength of the bone.

These materials are easily combined and printed by the use of 3D printing equipment. However, the reduced existence of biological signs to the cells, requires these matrices to be prepared with cells, to minimize the rejection process after implantation.

In this way, this invention largely considers the bone structure from the macro scale to the nano scale and presents the development of a three-dimensional structure having properties similar to that of the native tissue, the bone.

Apart from having biomimetic properties, the intelligent three-dimensional biomimetic structure presents magnetic properties, which makes it a structure that is responsive to internal stimulations (in the bone) or to external stimulations (such as magnetic fields).

Since they mimic the bone tissue in terms of composition, morphology, and mechanical and biological properties, the referred intelligent three-dimensional biomimetic structure becomes the most indicated choice in cases in which there is the need to regenerate the skeletal system following a trauma or cancer situation.

### SUMMARY OF THE INVENTION

In a first aspect, the present invention refers to an intelligent three-dimensional biomimetic structure comprising filaments comprised by:
- an organic phase comprising synthetic polymers, natural polymers or a mixture thereof, wherein said organic phase presents a concentration varying in the range of 10 to 70 % by weight;
- an inorganic phase comprising hydroxyapatite, wherein said inorganic phase presents a concentration varying in the range of 30 to 90 % by weight and wherein the hydroxyapatite is doped with ions which are incorporated within the crystalline structure thereof; and
wherein the polymers present in the organic phase are biomineralized with the doped hydroxyapatite present in the inorganic phase.

In a second aspect, it is a further object of the present invention the use of the intelligent three-dimensional biomimetic structure in the regeneration of the bone tissue, particularly subsequently to a trauma or cancer situation, by activating the particles by magnetic fields and release of ions contained therein for the prevention and treatment of diseases associated with the skeletal system.

### SOLUTION OF THE PROBLEM

This invention solves countless problems of the state of the art related to the three-dimensional models used in the treatment of diseases associated with bone degeneration.

A first point relates to the inflammation and rejection processes resulting from the use of bone substitutes, which, due to their characteristics, do not consider the specificities of the patient.

In the present invention, the intelligent three-dimensional biomimetic structure mimics the bone tissue in terms of composition, morphology, and mechanical and biological properties.

Additionally, the intelligent three-dimensional biomimetic structure is comprised by polymers mineralized with hydroxyapatite, which can further be doped with several ions. The doped hydroxyapatite is biomimetic and magnetic, and the presence of the different ions will grant antimicrobial and therapeutic properties.

Due to the countless properties thereof, the present invention contributes as an efficient and innovative alternative for bone regeneration.

### ADVANTAGEOUS EFFECTS OF THE INVENTION

The main advantage of the intelligent three-dimensional biomimetic structure described in the present invention is associated with the ability to mimic the bone tissue in terms of composition, morphology, and mechanical and biological properties.

Since it is comprised of synthetic or natural polymers mineralized with doped hydroxyapatite, said intelligent three-dimensional biomimetic structure presents magnetic, therapeutic, and anti-inflammatory properties, minimizing the inflammation and rejection processes.

Further, the doping of the hydroxyapatite can provide antimicrobial and therapeutic agents which are suitable for the intended use, whereby these are one more ally in the prevention, diagnosis and / or treatment of diseases associated with the skeletal system.

### BRIEF DESCRIPTION OF THE FIGURES

With the purpose of providing an understanding of the principles according to the embodiments of the present invention, reference will be made to the embodiments illustrated in the figures and to the terminology used to describe them.

It must further be understood that there is no intention of limiting the scope of the present invention to the content of the figures and that any subsequent alterations or modifications of the inventive characteristics shown herein, as well as any additional applications of the principles and embodiments of the invention shown, which would occur normally to a person skilled in the art having this description in hands, are considered as being within the scope of the claimed invention.
Figure 1 - illustrates the upper view of the intelligent three-dimensional biomimetic structure of this invention.
Figure 2 - illustrates the perspective view of the intelligent three-dimensional biomimetic structure of this invention.
Figure 3 - illustrates the perspective view of the intelligent three-dimensional biomimetic structure of this invention.
Figures 4A and 4B - illustrate the eccentric vertical longitudinal section view of the intelligent three-dimensional biomimetic structure of this invention.
Figures 5A and 5B - illustrate the eccentric horizontal longitudinal section view of the intelligent three-dimensional biomimetic structure of this invention

### DESCRIPTION OF THE EMBODIMENTS

The present invention refers to an intelligent three-dimensional biomimetic structure, as well as the use thereof in the regeneration of bone tissue.

Said intelligent three-dimensional biomimetic structure comprises filaments comprised by:
- an organic phase comprising synthetic polymers, natural polymers or a mixture thereof, wherein said organic phase presents a concentration varying in the range of 10 to 70 % by weight;
- an inorganic phase comprising hydroxyapatite, wherein said inorganic phase presents a concentration varying in the range of 30 to 90 % by weight and wherein the hydroxyapatite is doped with ions which are incorporated within the crystalline structure thereof; and
wherein the polymers present in the organic phase are biomineralized with the doped hydroxyapatite present in the inorganic phase.

Said intelligent three-dimensional biomimetic structure presents a geometry which mimics the bone tissue, in this manner providing the ability to regenerate the skeletal system subsequently to a trauma or cancer situation.

In a preferred embodiment of the present invention, the synthetic polymers are selected from the group consisting of polycaprolactone, poly lactic acid, poly lactic and glycolic acid among others, as well as combinations thereof.

In a preferred embodiment of the present invention, the natural polymers are selected from the group consisting of gelatin, collagen, recombinant peptides, among others, as well as combinations thereof.

The hydroxyapatite is additionally doped with ions selected from the group consisting of magnesium, strontium, iron, zinc, selenium, and combinations thereof.

The organic phase (which comprises the polymers of mixture of polymers) presents a concentration varying in the range of 10 to 70 % by weight, while the inorganic phase (which comprises the hydroxyapatite and ions) presents a concentration varying in the range of 30 to 90 % by weight.

The intelligent three-dimensional biomimetic structure of the invention, when utilizing natural polymers, is obtained by means of the production method already known to the state of the art and described in the documents "Bio-inspired polymeric iron-doped hydroxyapatite microspheres as a tunable carrier of rhBMP-2" (Patrício T. M. F. et al, 2021), "Superparamagnetic hybrid microspheres affecting osteoblasts behaviour" (Patrício T. M. F. et al, 2019) and "New bioactive bone-like microspheres with intrinsic magnetic properties obtained by bio-inspired mineralisation process" (Patrício T. M. F. et al, 2017).

The production method comprises the steps of:
a) Biomineralization; and
b) 3D printing.

The method optionally comprises a third phase:
c) Freeze drying.

In step (a), the materials are initially selected for the synthesis, wherein a solution of the natural polymer which constitutes the organic phase (acid pH) and another solution which constitutes the inorganic phase, comprising precursor ions for the formation of doped hydroxyapatite, (basic pH) are prepared.

The doping of the hydroxyapatite occurs concurrently with the biomineralization process, wherein the desired ions are added to the inorganic phase, thus constituting the precursor ions for the formation of the hydroxyapatite.

The organic phase is added drop by drop to the inorganic phase, reducing the pH progressively, maintaining the pH basic to neutral at the end of the reaction. The monitoring of the pH allows obtaining the materials with the desired properties, without obtaining new phases.

In step (b), the 3D printing of the mixture obtained by adding the organic phase to the inorganic phase allows the control of the architecture of the intelligent three-dimensional biomimetic structure, with the purpose of mimicking the bone tissue and improving the morphological, topographical, and mechanical properties of same.

Subsequently to the printing step, the intelligent three-dimensional biomimetic structure can be used directly, in the form of a hydrogel combined with cross-linking agents or, optionally, dry, in a subsequent freeze-drying step and which presents greater porosity.

When the polymer used is synthetic, the mineralization can be carried out in two manners:
- such as described above in step (a), wherein the biomineralization is carried out replacing the natural polymers with synthetic ones; or
- by the production of mineralized natural polymer particles with doped hydroxyapatite by means of the emulsification process.

The emulsification allows obtaining particles in nano or micro scale and which can be dense or porous. Subsequently, these particles can be mixed with the synthetic polymers, for subsequent manufacture of the intelligent three-dimensional biomimetic structure by 3D printing followed, if desired, by freeze drying.

The mechanical properties and the porosity can be combined with the natural and synthetic polymer matrix ratio, as well as the doped hydroxyapatite ratio. In this manner, the combination of the organic phase and the inorganic phase, as well as the geometry of the three-dimensional structure, will be matched to the desired application.

In this sense, the intelligent three-dimensional biomimetic structure of the present invention comprises biomineralized filaments comprised of:
- natural polymers mineralized with doped hydroxyapatite; and / or
- synthetic polymers mineralized with doped hydroxyapatite; and / or
- natural polymers mineralized with doped hydroxyapatite in the form of particles mixed with synthetic polymers, at different ratios of particles and synthetic polymers.

The geometry, the morphology, and the number of filaments of the intelligent three-dimensional biomimetic structure will depend on the place wherein it will be implanted.

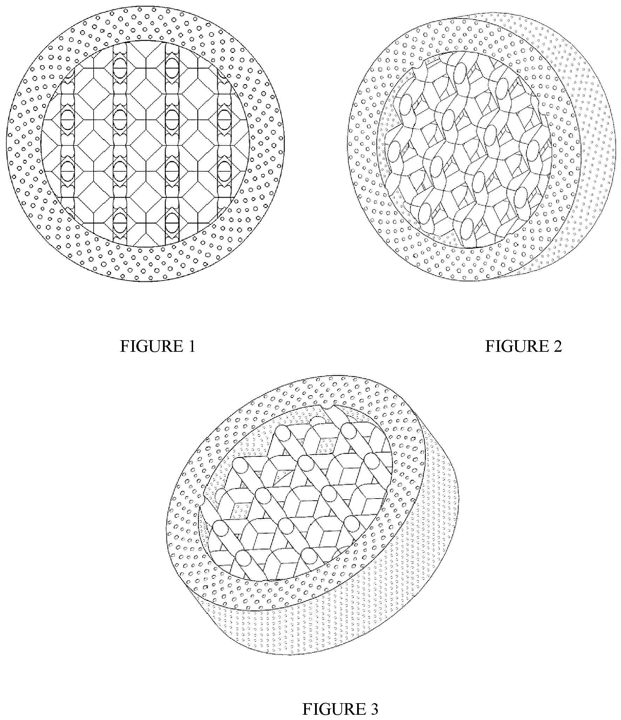

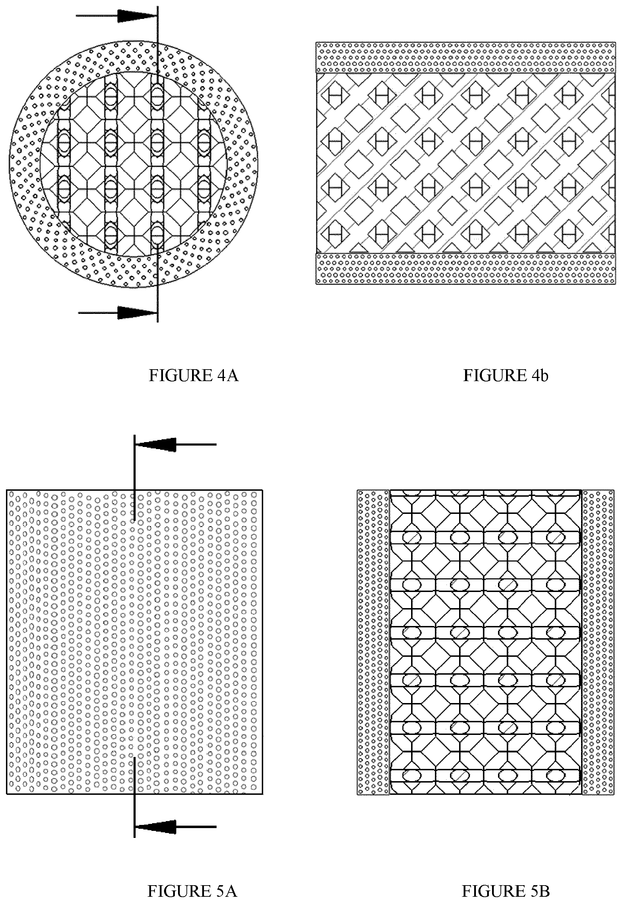

In a preferred embodiment of the present invention, said intelligent three-dimensional biomimetic structure has a cylindrical shape and can comprise longitudinal filaments in the outermost portion thereof and tangled fibers in its innermost portion (such as represented in Figures 1 to 5).

The intelligent three-dimensional biomimetic structure presents similar properties to that of the native tissues. This similarity with the native tissues is determined by the ratio between the organic and inorganic matrices, as well as by the inclusion of ions in the inorganic component of said structure.

The presence of ions, which are incorporated within the crystalline structure of the hydroxyapatite, can impart magnetic properties to the three-dimensional structure of this invention and can enhance the production of materials with therapeutic or antimicrobial properties.

On the other hand, the chemical composition of the inorganic matrix enables the chemical interaction with drugs or growth factors, providing a greater control in the bioactivity thereof, in the cases wherein it is necessary to improve the therapeutic properties. The inclusion of drugs or growth factors will be an added value, for example in the case of treatment of diseases.

The magnetic stimulation activates the doped hydroxyapatite, releasing the ions and remaining components present, apart from stimulating the activity and cell interaction, promoting an endogenous environment which allows the triggering of the cascade of phenomena which will lead to the tissue regeneration.

The geometry, the morphology, and the number of filaments of the intelligent three-dimensional biomimetic structure will depend on the place wherein it will be implanted. The number of filaments of the three-dimensional structure is influenced by the size and configuration of the lesion and can present micro scale dimensions up to ten centimeters in height and width.

Preferably, the geometry of the structure must follow the geometry of the bone with which it will interact. In the same manner, there must be a balance between the porosity and the mechanical properties of the structure and of the related bone.

The same occurs with the morphology: the arrangement of the filaments in the structure will match the arrangement of the collagen fibers in the bone with which the intelligent three-dimensional biomimetic structure will interact.

Further, the intelligent three-dimensional biomimetic structure proposed herein presents properties that are similar to those of the native tissue, with an adequacy between the degree of porosity, the mechanical properties, and the relevance of said characteristics at cell level.

In a preferred embodiment of this invention, the degree of porosity varies in the range of 10 - 95%, while the Young modulus varies in the range of 1 - 20 GPa.

The intelligent three-dimensional biomimetic structure of the present invention is used in the prevention, the diagnosis and the treatment of diseases associated with the skeletal system, by means of the activation of particles of doped hydroxyapatite through the magnetic fields and the release of therapeutic agents or ions contained therein.

In a preferred embodiment of the invention, the diseases associated with the skeletal system are selected from degenerative diseases, tumors, bone loss in case of need of dental implant and fractures, among others.

The object-matter described above is provided as an illustration of the present invention and, therefore, must not be interpreted to limit same. The terminology used with the purpose of describing preferred embodiments of the present invention must not be restricted to the same.

As used in the description, the definite and indefinite articles, in their singular form, aim at the interpretation of also including the plural forms, unless the context of the description explicitly indicates the opposite.

The indefinite article "one" must be interpreted generally as being "one or more", unless the meaning of a singular embodiment is clearly defined in a specific situation.

It will further be understood that the expressions "comprise" and "include", when used in this description, specify the presence of the characteristics, the elements, the components, the steps, and the related operations, however, they do not exclude the possibility of other characteristics, elements, components, steps, and operations also being contemplated.

As used throughout this patent application, the expression "or" is used in the inclusive sense instead of the exclusive sense, unless the exclusive sense is clearly defined in a specific situation. In this context, a sentence of the type "X uses A or B" must be interpreted as including all the inclusive pertinent combinations, for example "X uses A", "X uses B" and "X uses A and B".

All the alterations, providing that they do not modify the essential characteristics of the following claims, must be considered within the scope of protection of the present invention.

### LIST OF CITATIONS

Follows the list of citations:

### PATENT LITERATURE

- CN111110404A
- EP3266418B1
- EP2349107B1
- EP3563881A1

### NON-PATENT LITERATURE

- Patrício T. M. F., Mumcuogluv D., Montesi M., Panseri S., Witte-Bouma J., Garcia S. F., Sandri M., Tampieri A., Farrell E., Sprio S. (2021). Bio-inspired polymeric iron-doped hydroxyapatite microspheres as a tunable carrier of rhBMP-2, Materials Science and Engineering: C, Volume 119, 111410.
- Patrício T. M. F., Panseri S., Montesi M., Iafisco M., Sandri M., Tampieri A., Sprio S. (2019). Superparamagnetic hybrid microspheres affecting osteoblasts behaviour, Materials Science and Engineering: C, 96, pp. 234-247, doi.org/10.1016/j.msec.2018.11.014.
- Patrício, T. M. F., Panseri, S., Sandri, M., Tampieri, A., & Sprio, S. (2017). New bioactive bone-like microspheres with intrinsic magnetic properties obtained by bio-inspired mineralisation process. Materials Science and Engineering: C, 77, 613-623, DOI: 10.1016/j.msec.2017.03.258.

## Claims

1. Intelligent three-dimensional biomimetic structure **characterized by** comprising filaments comprised of:
- an organic phase comprising synthetic polymers, natural polymers or a mixture thereof, wherein said organic phase presents a concentration varying in the range of 10 to 70 % by weight;
- an inorganic phase comprising hydroxyapatite, wherein said inorganic phase presents a concentration varying in the range of 30 to 90 % by weight and wherein the hydroxyapatite is doped with ions which are incorporated within the crystalline structure thereof; and
wherein the polymers present in the organic phase are biomineralized with the doped hydroxyapatite present in the inorganic phase.

2. Intelligent three-dimensional biomimetic structure, according to claim 1, **characterized by** the synthetic polymers being selected from the group consisting of polycaprolactone, poly lactic acid, poly lactic and glycolic acid, and combinations thereof.

3. Intelligent three-dimensional biomimetic structure, according to claim 1, **characterized by** the natural polymers being selected from the group consisting in gelatin, collagen, recombinant peptides, and combinations thereof.

4. Intelligent three-dimensional biomimetic structure, according to claim 1, **characterized by** the ions being selected from the group consisting of magnesium, strontium, iron, zinc, selenium, and combinations thereof.

5. Intelligent three-dimensional biomimetic structure, according to claim 1, **characterized by** the inorganic phase comprising components selected from the group consisting of cells, drugs, and growth factors.

6. Intelligent three-dimensional biomimetic structure, according to any one of claims 1 to 5, **characterized by** the geometry following the geometry of the bone with which the intelligent three-dimensional biomimetic structure will interact.

7. Intelligent three-dimensional biomimetic structure, according to any one of claims 1 to 6, **characterized by** the arrangement of the filaments following the arrangement of the collagen fibers in the bone with which the intelligent three-dimensional biomimetic structure will interact.

8. Intelligent three-dimensional biomimetic structure, according to any one of claims 1 to 7, **characterized by** the number of filaments varying according to the size and the configuration of the lesion, wherein the dimensions vary in micro scale up to ten centimeters in height and width.

9. Intelligent three-dimensional biomimetic structure, according to any one of claims 1 to 8, **characterized by** the porosity degree being between 10 - 95 %.

10. Intelligent three-dimensional biomimetic structure, according to any one of claims 1 to 9, **characterized by** the Young modulus varying in the range of 1 - 20 GPa.

11. Use of the intelligent three-dimensional biomimetic structure as defined in claims 1 to 10, **characterized by** being in the activation of the particles by magnetic fields and release of the therapeutic agents or ions contained therein for the prevention and the treatment of diseases associated with the skeletal system.

12. Use of the intelligent three-dimensional biomimetic structure, according to claim 11, **characterized by** the diseases associated with the skeletal system being selected from degenerative diseases, tumors, bone loss in case of need of dental implant and fractures.
